# EUROPÄISCHE PATENTSCHRIFT

(11) **EP 0 858 318 B1**
(45) Veröffentlichungstag und Bekanntmachung des Hinweises auf die Patenterteilung: **06.06.2001**
(21) Anmeldenummer: 96937228.3
(22) Anmeldetag: 25.10.1996
(51) Int. Cl.: A61K 7/42

(54) **PHOTOSTABILE UV-A-FILTER ENTHALTENDE KOSMETISCHE ZUBEREITUNGEN**
COSMETIC PREPARATIONS CONTAINING PHOTOSTABLE UV-A FILTERS
PREPARATIONS COSMETIQUES RENFERMANT DES ECRANS UV-A PHOTOSTABLES

(30) Priorität: 03.11.1995 DE 19540952
(43) Veröffentlichungstag der Anmeldung: 19.08.1998
(73) Patentinhaber: BASF AKTIENGESELLSCHAFT, 67056 Ludwigshafen (DE)
(72) Erfinder: HOLDERBAUM, Martin, D-67065 Ludwigshafen (DE); AUMÜLLER, Alexander, D-67435 Neustadt (DE); SPERLING, Karin, D-67433 Neustadt (DE); WESTENFELDER, Horst, D-67435 Neustadt (DE); WÜNSCH, Thomas, D-67346 Speyer (DE)
(86) Internationale Anmeldenummer: EP9604637
(87) Internationale Veröffentlichungsnummer: WO9717054

(56) Entgegenhaltungen:
- DE-B- 1 242 780
- FR-A- 1 309 169
- US-A- 3 270 045
- US-A- 3 275 520

## Beschreibung

Die Erfindung betrifft die Verwendung von substituierten Diphenylmalonsäuredinitrilen als photostabile UV-A-Filter in kosmetischen Zubereitungen zum Schutz der menschlichen Epidermis gegen UV-Strahlung, speziell im Bereich von 320 bis 400 nm.

Die in kosmetischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschuztmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARASOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP 0514491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Es bestand daher die Aufgabe, Lichtschutzmittel für kosmetische Zwecke vorzuschlagen, die im UV-A-Bereich absorbieren und die photostabil sind.

Diese Aufgabe wurde erfindungsgemäß gelöst mit dem Zusatz von Verbindungen der Formel I 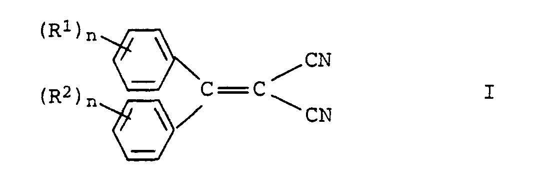 in der R¹ und R² in para und/oder ortho-Stellung stehende gleiche oder verschiedene geradkettige oder verzweigte aliphatische oder cycloaliphatische Reste mit 1 bis 18 C-Atomen bedeuten und wobei R¹ zusätzlich ein Wasserstoffatom bedeuten kann, und wobei ferner n die Zahlen 1 oder 2 bedeutet, sofern diese Verbindungen ihre wesentliche Absorption im Bereich von 320 bis 380 nm haben, als UV-A-Filter in kosmetischen Zubereitungen zum Schutz der menschlichen Haut gegen Sonnenstrahlen zusammen mit an sich für kosmetische Zubereitungen bekannten, im UV-B-Bereich absorbierenden Verbindungen.

Unter aliphatischen und cycloaliphatischen Resten werden vor allem gegebenenfalls durch Sauerstoffatome unterbrochene Kohlenwasserstoffreste verstanden.

Dabei ist die Verwendung von Verbindungen der Formel I bevorzugt 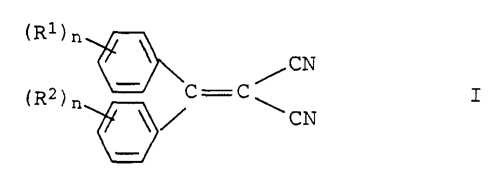 in der R¹ und R² gleich oder verschieden sind und geradkettige oder verzweigte Alkylreste mit 1 bis 18 Kohlenstoffatomen, bei denen die Alkylkette durch Sauerstoffatome unterbrochen sein kann, bedeuten und wobei R¹ auch Wasserstoff bedeuten kann und n für die Zahlen 1 und 2 steht.

Insbesondere kommen solche Verbindungen der Formel I in Betracht, in der R¹ und R² gleiche oder verschiedene in para-Stellung stehende verzweigte Alkoxyreste mit 3 bis 12 C-Atomen bedeutet und n jeweils für den Wert 1 steht und R¹ auch Wasserstoff bedeuten kann.

Besonders bevorzugt sind die folgenden Einzelverbindungen, in der R¹ und/oder R² in para-Stellung stehen und

| | |
|---|---|
| n-propoxy- | isopropoxy- |
| n-butoxy- | 1-methylpropoxy- |
| 2-methylpropoxy- | n-pentoxy- |
| 1,1-dimethylpropoxy- | 3-methylbutoxy- |
| hexoxy- | 2,2-dimethylpropoxy- |
| heptoxy- | 1-methyl-1-ethylpropoxy- |
| 2-ethylhexoxy- | und/oder octoxy- bedeuten |

und wobei R¹ auch Wasserstoff bedeuten kann.

Die erfindungsgemäß zu verwendenden Verbindungen werden zweckmäßig in an sich bekannter Weise nach einem Verfahren gemäß Georges Charles, Bull.Soc.Chim. 1962, S. 1559, hergestellt, bei dem man anstelle von Benzophenon als Ausgangsverbindungen die entsprechenden Imine der Formel II 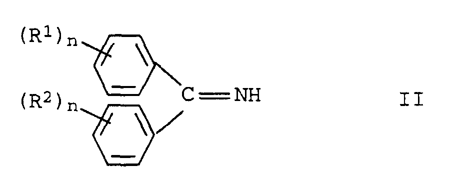 in der R¹ und R² und n die oben angegebenen Bedeutungen haben, mit Malonsäuredinitril in polaren organischen Lösungsmitteln unter Wasserausschluss umsetzt.

Dieses Verfahren hat mehrere Vorteile: es wird kein Katalysator benötigt; die Ausbeuten sind besser und die Reaktion läuft bei niedrigeren Temperaturen ab. Die Ausgangsverbindungen der Formel II können z.B. nach dem Verfahren der DE-A 4442138 erhalten werden.

Die Lichtschutzmittel enthaltenden kosmetischen Zubereitungen sind in der Regel auf der Basis eines Trägers, der mindestens eine Ölphase enthält. Es sind aber auch Zubereitungen allein auf wässriger (Gel)Basis möglich. Demgemäß kommen Öle, Öl-in-Wasser-und Wasser-in-Öl-Emulsionen, Cremes und Pasten, Lippenschutzstiftmassen oder Gele in Betracht.

Solche Sonnenschutzpräparate können demgemäß in flüssiger, pastöser oder fester Form vorliegen, beispielsweise als Waser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Emulgatoren wie Fettalkoholethoxylate, Sorbitanfettsäureester oder Lanolinderivate, Verdickungsmittel wie Carboxymethylcellulose oder vernetzte Polyacrylsäure, Konservierungsmittel und Parfüms. Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Gegenstand der vorliegenden Erfindung sind weiterhin kosmetische Zubereitungen, die 0,1 bis 10 Gew.-%, vorzugsweise 1 bis 7 Gew.-%, bezogen auf die gesamte Menge der kosmetischen Zubereitung, eine oder mehrere der Verbindungen der Formel I zusammen mit an sich für kosmetische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Der größte Teil der Lichtschutzmittel in den zum Schutz der menschlichen Epidermis dienenden kosmetischen Zubereitungen besteht aus Verbindungen, die UV-Licht im UV-B-Bereich absorbieren d.i. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-B-Filtersubstanzen, die in Kombination mit den erfindungsgemäß zu verwendenden Verbindungen der Formel I angewandt werden, kommen beliebige UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'- (1,4-Phenylendi-methin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 7/6/7-10-2 |
| 11 | 4-Methoxy-zimtsäure-2-ethylhexylester | 5466-77-3 |
| 12 | 2-Hydroxy-4-methoxy-benzophenon-5-sulfon(Sulisobenzonum) und das Natriumsalz | 4065-45-6 |
| 13 | 3-(4'-Sulfo)benzyliden-bornan-2-on und Salze | 58030-58-6 |
| 14 | 3-(4'-Methyl)benzyliden-bornan-2-on | 36861-47-9 |
| 15 | 3-Benzylidenbornan-2-on | 16087-24-8 |
| 16 | 1-(4'-Isopropylphenyl)-3-phenylpropan-1,3-dion | 63260-25-9 |
| 17 | 4-Isopropylbenzylsalicylat | 94134-93-7 |
| 18 | 2,4,6-Trianilin-(o-carbo-2'-ethylhexyl-1'-oxy)-1,3,5-triazin | 88122-99-0 |
| 19 | 3-Imidazol-4-yl-acrylsäure und ihr Ethylester | 104-98-3* |
| 20 | 2-Cyano-3,3-diphenylacrylsäureethylester | 5232-99-5 |
| 21 | 2-Cyano-3,3-diphenylacrylsäure-2'-ethylhexylester | 6197-30-4 |
| 22 | Menthyl-o-aminobenzoate oder: 5-Methyl-2-(1-methylethyl)-2-aminobenzoate | 134-09-8 |
| 23 | Glyceryl p-aminobenzoat oder: 4-Aminobenzoesäure-1-glyceryl-ester | 136-44-7 |
| 24 | 2,2'-Dihydroxy-4-methoxybenzophenon (Dioxybenzone) | 131-53-3 |
| 25 | 2-Hydroxy-4-methoxy-4-methylbenzophenon (Mexonon) | 1641-17-4 |
| 26 | Triethanolamin Salicylat | 2174-16-5 |
| 27 | Dimethoxyphenylglyoxalsäure oder: 3,4-dimethoxy-phenyl-glyoxal-saures Natrium | |
| 28 | 3-(4'Sulfo)benzyliden-bornan-2-on und seine Salze | 56039-58-8 |

Schließlich sind auch mikronisierte Pigmente wie Titandioxid und Zinkoxid zu nennen.

Die erfindungsgemäß zu verwendenden Verbindungen zeichnen sich ir der Regel durch ein besonders hohes Absorptionsvermögen im Bereich der UV-A-Strahlung aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Aus US 3,270,045 (1966) ist bekannt, Verbindungen der Formel 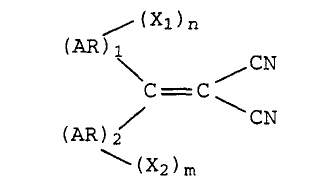 in der AR einen aromatischen Rest und X₁ auch einen Alkoxyrest bedeuten als Lichtschutzmittel für die Stabilisierung von organischen Materialien selbst, die durch UV-Strahlung verändert werden, insbesondere Kunststoffe zu verwenden. Unter der großen Zahl von gegen UV-Strahlung zu stabilisierenden Substanzen, wie Plastikmaterialien, Folien, geschäumte Materialien, deren Oberfläche geschützt werden soll, sind auch "polishes, creams, lotions and the like" genannt. Dem gesamten Kontext dieser Patentschrift kann jedoch der Fachmann nicht die Lehre der vorliegenden Erfindung entnehmen, d.h. (a) diese Verbindungen als Lichtschutzmittel für die menschliche Haut zu verwenden, (b) eine Auswahl in Bezug auf UV-A-Absorber zu treffen und (c) diese in Kombination mit an sich für kosmetische Zubereitungen bekannten im UV-B-Bereich absorbierenden Verbindungen zu verwenden.

Dies umsomehr, als diese Literaturstelle aus dem Jahr 1966 stammt und, wie eingangs erläutert, bisher keine stabilen UV-A-Absorber für die kosmetische Anwendung zur Verfügung standen, vielmehr die Entwicklung in Richtung einer externen Stabilisierung von allein nicht stabilen UV-A-Absorbern lief.

### Herstellung

### Beispiel 1

Allgemeine Vorschrift:
0,2 mol alkyliertes Benzophenon wird in 200 ml Heptan mit einer äquimolaren Menge an Malodinitril und 20 ml einer Katalysatormischung aus Amoniumacetat/Eisessig (Molverhältnis 1:4) versetzt und 20 h am Wasserabscheider unter Rückfluß gekocht. Während der Reaktion werden stündlich weitere 3 ml der Katalysatormischung zudosiert. Anschließend wird der restliche Katalysator bei ca. 50°C abgetrennt und auf Raumtemperatur abgekühlt. Die Produkte fallen aus. Danach saugt man ab und kristallisiert mehrmals aus Essigester um. Als Öl anfallende Produkte werden über eine Kieselgelsäule mit Methylenchlorid als Laufmittel gereinigt (Ausbeute: 35 %). Die einzelnen hergestellten Stoffe sind in Tabelle 1 angegeben.

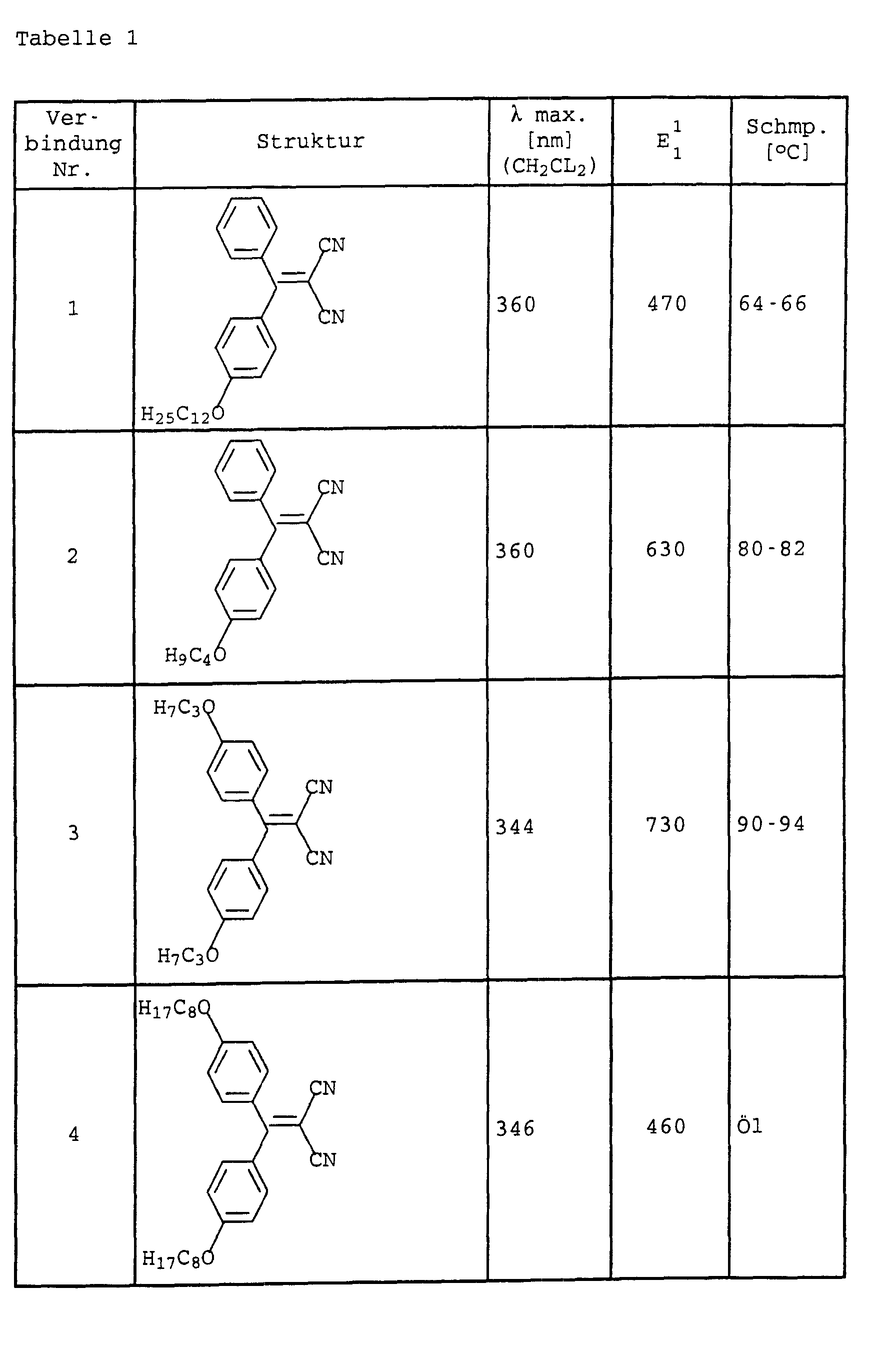

Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 2

| Zusammensetzung für die Lippenpflege | |
|---|---|
| ad 100 | Eucerinum anhydricum |
| 10,00 | Glycerin |
| 10,00 | Titanium Dioxid |
| 0,5-10 | Verbindung der Tabelle 1, Nr. 2 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3

| Zusammensetzung für Sunblocker mit Mikropigmenten | |
|---|---|
| ad 100 | Wasser |
| 10,00 | Octyl Methoxcinnamat |
| 6,00 | PEG-7-Hydrogenated Castor Öl |
| 6,00 | Titanium Dioxid |
| 0,5-10 | Verbindung der Tabelle 1, Nr. 4 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Butyl Methoxydibenzoylmethan |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 4

| Fettfreies Gel | |
|---|---|
| ad 100 | Wasser |
| 8,00 | Octyl Methoxycinnamat |
| 7,00 | Titanium Dioxid |
| 0,5-10 | Verbindung der Tabelle 1, Nr. 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 5

| Sonnencreme (LSF 20) | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 0,5-10 | Verbindung der Tabelle 1, Nr. 2 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 6

| Sonnencreme wasserfest | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Verbindung nach Tabelle 1, Nr. 3 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 7

| Sonnenmilch (LSF 6) | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 0,5-10 | Verbindung nach Tabelle 1, Nr. 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von Verbindungen der Formel I 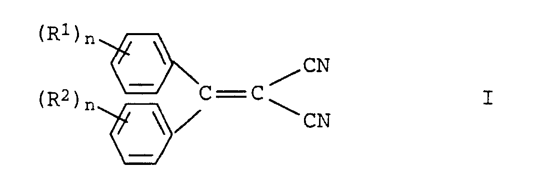 in der R¹ und R² in para und/oder ortho-Stellung stehende gleiche oder verschiedene geradkettige oder verzweigte aliphatische oder cycloaliphatische Reste mit 1 bis 18 C-Atomen bedeuten und wobei R¹ zusätzlich ein Wasserstoffatom bedeuten kann, und wobei ferner n die Zahlen 1 oder 2 bedeutet, als UV-A-Filter in kosmetischen Zubereitungen zum Schutz der menschlichen Haut gegen Sonnenstrahlen, zusammen mit an sich für kosmetische Zubereitungen bekannten, im UV-B-Bereich absorbierenden Verbindungen.

2. Verwendung gemäß Anspruch 1 von Verbindungen der Formel I 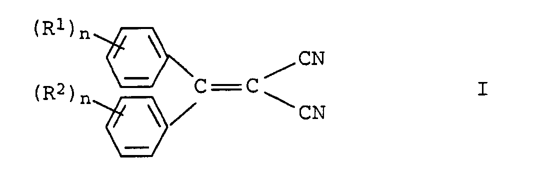 in der R¹ und R² gleich oder verschieden sind und geradkettige oder verzweigte Alkylreste mit 1 bis 18 Kohlenstoffatomen, bei denen die Alkylkette durch Sauerstoffatome unterbrochen sein kann, bedeuten und wobei R¹ auch Wasserstoff bedeuten kann und n für die Zahlen 1 und 2 steht.

3. Verwendung gemäß Anspruch 1 von Verbindungen der Formel I, in der R¹ und R² gleiche oder verschiedene in para-Stellung stehende verzweigte Alkoxyreste mit 3 bis 12 C-Atomen bedeutet und n jeweils für den Wert 1 steht und R¹ außerdem Wasserstoff bedeuten kann.

4. Lichtschutzmittel enthaltende kosmetische Zubereitungen zum Schutz der menschlichen Epidermis gegen UV-Licht im Bereich von 280 bis 400 nm, dadurch gekennzeichnet, daß sie in einem kosmetisch geeigneten Träger, zusammen mit an sich für kosmetische Zubereitungen bekannten im UV-B-Bereich absorbierenden Verbindungen, als photostabile UV-A-Filter wirksame Mengen von Verbindungen der Formel I gemäß Anspruch 1 enthalten, in der R¹ und R² und n die in Anspruch 1 angegebenen Bedeutungen haben.

5. Lichtschutzmittel enthaltende kosmetische Zubereitungen gemäß Anspruch 4, enthaltend als UV-A-Filter Verbindungen der Formel I gemäß Anspruch 1, in der R¹ und R² gleiche oder verschiedene Alkoxyreste mit 1 bis 18 C-Atomen bedeuten und n die Zahlen 1 oder 2 bedeutet und R¹ auch ein Wasserstoffatom bedeuten kann.

6. Lichtschutzmittel enthaltende kosmetische Zubereitungen gemäß Anspruch 4, in denen der kosmetisch geeignete Träger mindestens eine Ölphase enthält.

## Claims

1. The use of compounds of the formula I 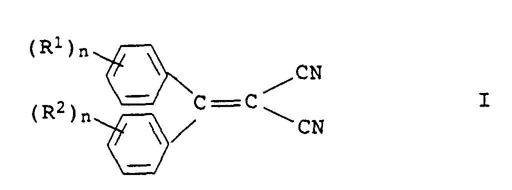 where R¹ and R² are identical or different straight-chain or branched aliphatic or cycloaliphatic radicals which are in the para and/or ortho position and have 1 to 18 carbon atoms, and where R¹ may additionally be a hydrogen atom, and where furthermore n is 1 or 2, as UV-A filters in cosmetic formulations to protect the human skin from the sun's rays, together with compounds which absorb in the UV-B region and are known per se for cosmetic formulations.

2. The use as claimed in claim 1 of compounds of the formula I 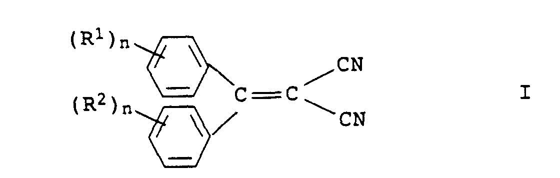 where R¹ and R² are identical or different and are straight-chain or branched alkyl radicals having 1 to 18 carbon atoms, in which the alkyl chain can be interrupted by oxygen atoms, and where R¹ can also be hydrogen, and n is 1 and 2.

3. The use as claimed in claim 1 of compounds of the formula I, where R¹ and R² are identical or different branched alkoxy radicals which are in the para position and have 3 to 12 carbon atoms, and n is in each case 1, and R¹ can additionally be hydrogen.

4. A sunscreen-containing cosmetic formulation to protect the human epidermis from UV light in the range from 280 to 400 nm, which comprises in a cosmetically suitable carrier, together with compounds which absorb in the UV-B region and are known per se for cosmetic formulations, as photostable UV-A filters effective amounts of compounds of the formula I as claimed in claim 1, where R¹ and R² and n have the meanings stated in claim 1.

5. A sunscreen-containing cosmetic formulation as claimed in claim 4, comprising as UV-A filters compounds of the formula I as claimed in claim 1, where R¹ and R² are identical or different alkoxy radicals having 1 to 18 carbon atoms, and n is 1 or 2, and R¹ can also be a hydrogen atom.

6. A sunscreen-containing cosmetic formulation as claimed in claim 4, wherein the cosmetically suitable carrier comprises at least one oil phase.

## Revendications

1. Utilisation de composés de formule I 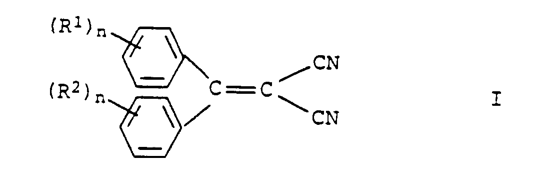 dans laquelle R¹ et R² représentent des radicaux aliphatiques à chaîne droite ou ramifiée ou cycloaliphatiques en C1-C18 identiques ou différents en positions para et/ou ortho, R¹ pouvant en outre représenter un atome d'hydrogène, et n est égal à 1 ou 2, en tant que filtres des UV-A dans des compositions cosmétiques destinées à protéger la peau humaine contre les rayons solaires, avec des composés absorbant dans le domaine UV-B et connus en soi pour des compositions cosmétiques.

2. Utilisation selon la revendication 1 de composés de formule I 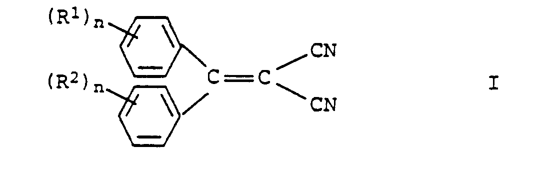 dans laquelle R¹ et R², ayant des significations identiques ou différentes, représentent des groupes alkyle à chaîne droite ou ramifiée en C1-C18 dont les chaînes peuvent être interrrompues par des atomes d'oxygène, R¹ pouvant également représenter l'hydrogène, et n est égal à 1 ou 2.

3. Utilisation selon la revendication 1 de composés de formule I dans laquelle R¹ et R², ayant des significations identiques ou différentes, représentent des groupes alcoxy à chaîne ramifiée en C3-C12 en position para, n est égal à 1 dans les deux cas et R¹ peut en outre représenter l'hydrogène.

4. Compositions cosmétiques contenant des agents protégeant contre la lumière et visant à la protection de l'épiderme humain contre la lumière UV dans l'intervalle de 280 à 400 nm, caractérisées par le fait qu'elles contiennent, dans un véhicule approprié aux usages cosmétiques, avec des composés absorbant dans le domaine UV-B et connus en soi pour des compositions cosmétiques, des composés de formule I selon la revendication 1 dans laquelle R¹ et R² et n ont les significations indiquées dans la revendication 1, en quantités efficaces en tant que fibres photostables pour les UV-A.

5. Compositions cosmétiques contenant des agents de protection contre la lumière selon la revendication 4, qui contiennent, en tant que filtres pour les UV-A, des composés de formule I selon la revendication 1 dans laquelle R¹ et R², ayant des significations identiques ou différentes, représentent des groupes alcoxy en C1-C18, n est égal à 1 ou 2, R¹ pouvant également représenter un atome d'hydrogène.

6. Compositions cosmétiques contenant des agents de protection contre la lumière selon la revendication 4, dans lesquelles le véhicule approprié aux usages cosmétiques contient au moins une phase huileuse.
